# DEMANDE DE BREVET EUROPEEN

(11) **EP 0 744 398 A1**
(43) Date de publication de la demande: **27.11.1996**
(21) Numéro de dépôt: 96401031.8
(22) Date de dépôt: 13.05.1996
(51) Int. Cl.: C07C 319/00, C10M 135/04

(54) **Hydrocarbures éthyléniques sulfurés par le soufre élémentaire en présence de carbonate de guanidine, leur préparations et leurs utilisations**

(30) Priorité: 24.05.1995 FR 9506348
(71) Demandeur: INSTITUT FRANCAIS DU PETROLE, 92502 Rueil-Malmaison (FR)
(72) Inventeur: Delfort, Bruno, 75005 Paris (FR); Lacome, Thierry, 92500 Rueil Malmaison (FR); Born, Maurice, 92000 Nanterre (FR)

(57) **Abrégé**

On décrit des produits soufrés dérivant d'hydrocarbures mono ou polyéthyléniques par sulfuration au moyen de soufre élémentaire en présence de carbonate de guanidine et en présence d'eau.

Les produits soufrés, qui peuvent contenir de 20 à 70 % en masse de soufre, sont généralement solubles dans les lubrifiants, dont ils améliorent les propriétés antiusure et extrême-pression. Ils peuvent également être utilisés comme agents de sulfuration notamment dans la préparation de catalyseurs de raffinage de produits pétroliers.

## Description

L'invention concerne de nouveaux produits organiques soufrés, un procédé pour leur préparation et leurs utilisations.

Divers produits organiques soufrés sont utilisés depuis longtemps comme additifs dans les lubrifiants pour améliorer notamment les propriétés antiusure et extrême-pression. Il peut s'agir notamment de lubrifiants pour moteurs, pour engrenages fortement chargés ou de lubrifiants pour le travail des métaux.

Comme additifs antiusure et extrême-pression, ont été largement décrits des produits organiques soufrés obtenus par divers procédés comprenant principalement deux étapes :
1) la formation d'un produit d'addition entre le chlorure de soufre (mono ou dichlorure de soufre) et un composé à insaturation éthylénique, et
2) la réaction de ce produit d'addition avec un composé sulfuré : sulfure, hydrosulfure ou polysulfure de métal alcalin (par exemple de sodium), mis en jeu tels que ou formés in situ par réaction d'hydrogène sulfuré (H₂S) ou de mercaptans avec un hydroxyde de métal alcalin (par exemple la soude).

Pour accroître la teneur en soufre des produits obtenus, il était possible de mettre en jeu du soufre élémentaire au cours de la seconde étape décrite ci-dessus.

Du fait de la mise en jeu de composés chlorés dans la première étape de leur préparation, les produits soufrés de ce type contiennent toujours une certaine quantité résiduelle de chlore, qui a pu être réduite à moins de 250 ppm par diverses amélioration apportées au procédé, notamment par la demandresse, ces derniers années. De plus, ces procédés engendrent la formation, comme sous produits, de chlorure de métal alcalin (en général NaCl) et de dérivés organiques alcalins hydrosolubles, que l'on retrouve sous la forme d'une solution aqueuse très polluante, produite en quantité très importante et, de ce fait, très coûteuse à éliminer.

On connaît par ailleurs divers procédés de préparation de produits soufrés ne mettant pas en jeu de composés chlorés. Certains de ces procédés sont basés sur la sulfuration des oléfines par le soufre élémentaire et l'hydrogène sulfuré (H₂S). Ces procédés présentent l'inconvénient de mettre en jeu un réactif extrêmement toxique, difficile à stocker et à transporter et de développer des pressions généralement élevées qui peuvent aller par exemple jusqu'à 90 bars.

D'autres procédés mettent en jeu du soufre élémentaire et des hydrosulfures alcalins ou alcalino-terreux.

On a maintenant découvert qu'il était possible de préparer des produits soufrés à partir d'hydrocarbures mono ou polyéthyléniques en utilisant du soufre élémentaire comme seul réactif soufré, en opérant en présence de carbonate de guanidine de formule : 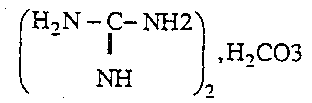 et d'eau.

Les produits soufrés de l'invention sont exempts de chlore, ils présentent en général une teneur en soufre pouvant aller d'environ 20 à 70 % en masse selon l'hydrocarbure éthylénique de départ. Les produits soufrés de l'invention sont solubles à des degrés divers dans les huiles minérales et dans les polyalphaoléfines hydrogénées, même - ce qui est tout à fait inattendu - les produits ayant une teneur en soufre élevée.

Les produits soufrés de l'invention peuvent être définis d'une manière générale par le fait qu'ils sont obtenus par réaction d'au moins un hydrocarbure mono ou polyéthylénique renfermant en général de 2 à 36 atomes de carbone et de 1 à 3 insaturations éthyléniques avec une quantité appropriée de soufre élémentaire en présence de carbonate de guanidine et d'eau.

Les hydrocarbures mono ou polyéthyléniques de départ peuvent être à chaîne ouverte, linéaire ou ramifiée, ou cycliques. Comme exemples, on peut citer : l'éthylène, le propylène, le 1-butène, les n-butènes-2, l'isobutène, les divers pentènes, les divers hexènes (par exemple le 2,3 -diméthyl 1- butène) ainsi que le 1, 3-butadiène, l'isoprène, le cyclopentadiène, le dicyclopentadiène, les trimères et tétramères du propylène, les trimères et tétramères de l'isobutène, ainsi que les polyalphaoléfines non hydrogénées de faible masse molaire (allant par exemple jusqu'à environ 500). On utilise de préférence l'isobutène.

Dans la réaction de préparation des produits soufrés de l'invention, on met en jeu une proportion de soufre élémentaire allant jusqu'à environ 250 g (soit environ 8 moles) par insaturation éthylénique dans l'hydrocarbure de départ.

On introduit également dans le milieu de réaction de l'eau en une proportion pouvant varier par exemple de 50 à 500 cm3 par insaturation éthylénique de l'hydrocarbure de départ.

La proportion de carbonate de guanidine mise en jeu peut aller de 3.10⁻³ à 1,2 mole par insaturation éthylénique de l'hydrocarbure de départ.

On remarque avec surprise que le carbonate de guanidine peut être utilisé notamment en très faible proportion (quasi catalytique).

En général, la réaction est réalisée à une température de 50 à 200°C et le plus souvent de 90 à 160°C.

La pression, qui dépend de manière prépondérante de la nature du ou des hydrocarbures mono ou polyéthyléniques de départ, peut aller de la pression atmosphérique jusqu'à par exemple 50 bars. Ainsi, pour les hydrocarbures éthyléniques gazeux dans les conditions normales, la pression s'établit entre 10 et 50 bars selon la proportion entre l'hydrocarbure éthylénique et le carbonate de guanidine. La durée de la réaction est par exemple de 0,5 à 24 heures.

Les produits de l'invention sont des produits liquides renfermant généralement de 20 à 70 % en masse de soufre ; ils sont limpides et homogènes même aux hautes teneurs en soufre. Leur coloration varie selon la nature de l'hydrocarbure de départ et la quantité de soufre fixée. Ils peuvent être de très peu ou peu colorés (dans le cas par exemple des hydrocarbures monoéthyléniques soufrés tels que l'isobutène soufré) à très colorés (dans le cas par exemple des hydrocarbures polyéthyléniques soufrés).

Dans le cas des hydrocarbures monoéthyléniques, les produits soufrés se caractérisent par la quasi absence d'atomes de carbone éthyléniques.

Les teneurs en soufre des produits de l'invention leur conférent des propriétés antiusure et extrême-pression telles qu'ils peuvent utilisés avantageusement comme additifs dans les huiles et graisses lubrifiantes, notamment dans la formulation d'huiles pour engrenages d'automobiles et d'huiles industrielles.

Pour ces utilisations, on incorpore en général les produits de l'invention aux compositions lubrifiantes à des concentrations de 0,05 à 20 % de préférence de 0,5 à 10 % en masse.

Certains des produits soufrés de l'invention, notamment ceux qui dérivent de la sulfuration de l'isobutène, peuvent encore être utilisés comme agents de sulfuration en particulier dans la préparation de catalyseurs de raffinage de produits pétroliers.

Les exemples suivants illustrent l'invention.

### Exemple 1

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 63g (1,97 mole) de soufre, 29,3g (0,163 mole) de carbonate de guanidine, 222,5 g d'eau et 36g (0,642 mole) d'isobutène. On porte le milieu à la température de 130°C pendant 10 heures. La pression maximale atteinte est de 28 bars. Après retour à la température ambiante, on extrait le milieu avec 150 ml de n-heptane. Après lavage à la soude à 10% en masse, puis lavage à l'eau, on évapore le n-heptane sous pression réduite et on recueille 75g d'un liquide limpide dont la teneur en soufre est de 56,9% en masse.

### Exemple 2

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 75g (2,34 mole) de soufre, 29,3g (0,163 mole) de carbonate de guanidine, 135g d'eau, 44,8g (0,77 mole) d'isobutène et 2,6g (0,046 mole) de 1,3-butadiène. On porte le milieu à la température de 130°C pendant 9 heures. La pression maximale atteinte est de 29 bars. Après retour à la température ambiante, on extrait le milieu avec 150 ml de n-heptane. Après lavage à la soude à 10% en masse, puis lavage à l'eau, on évapore le n-heptane sous pression réduite et on recueille 68g d'un liquide limpide dont la teneur en soufre est de 53 % en masse.

### Exemple 3 : Examen de la solubilité des produits de l'invention

Les produits de l'invention préparés comme décrits dans les exemples 1 et 2 sont examinés pour leur solubilité dans une huile minérale 130 Neutral Solvent et dans une haute synthétique de type polyalphaoléfine hydrogènée (PAO 6), à une concentration permettant d'ajuster la teneur en soufre à 2% en masse. Dans tous les cas, les produits sont solubles.

L'examen par C13 RMN des produits préparés dans les exemples 1 et 2 indique l'absence d'atomes de carbones éthyléniques.

### Exemple 4 : Caractérisation des propriétés extrême-pression

Les produits de l'invention préparés comme décrits dans les exemples 1 et 2, ci-dessus sont caractérisés pour leurs propriétés extrême-pression dans une huile minérale 130 Neutral Solvent à une concentration permettant d'ajuster la concentration en soufre due à l'additif à 2% en masse. La caractérisation est effectuée au moyen d'une machine 4 billes selon l'essai ASTM D2783. Les résultats sont les suivants.

| | | | **Essais sur machine 4 billes** | |
|---|---|---|---|---|
| **Produit de l'ex n°** | **% en masse additif/huile** | **% en masse S/Huile** | **Charge de soudure (daN)** | **Indice Charge/Usure (daN)** |
| 1 | 3,51 | 2 | 440 | 83 |
| 2 | 3,77 | 2 | 410 | 75 |

## Revendications

1. Produit soufré caractérisé en ce qu'il est obtenu par réaction d'au moins un hydrocarbure mono ou polyéthylénique renfermant de 2 à 36 atomes de carbone et de 1 à 3 insaturations éthyléniques avec du soufre élémentaire en présence de carbonate de guanidine et d'eau .

2. Produit soufré selon la revendication 1, caractérisé en ce l'hydrocarbure mono ou polyéthylénique est choisi parmi l'éthylène, le propylène, le butène-1, les n-butènes-2, l'isobutène, les divers pentènes, les divers hexènes ainsi que le 1, 3-butadiène, l'isoprène, le cyclopentadiène, le dicyclopentadiène des dimères et trimères de l'isobutène, les trimères et tétramères du propylène, ainsi que les polyalphaoléfines non hydrogénées de masse molaire allant jusqu'à environ 500.

3. Produit soufré selon la revendication 1 ou 2, caractérisé en ce que ledit hydrocarbure est l'isobutène.

4. Produit soufré selon la revendication 1 ou 2, caractérisé en ce que ledit hydrocarbure est un mélange d'isobutène et de 1,3 butadiène.

5. Produit soufré selon l'une revendications 1 à 4, caractérisé en ce que l'on met en jeu une proportion de soufre élémentaire allant jusqu'à environ 250g (soit environ 8 moles) par insaturation éthylénique de l'hydrocarbure de départ.

6. Produit soufré selon l'une des revendications 1 à 5, caractérisé en ce que l'on met en jeu une proportion de 50 à 500 cm3 par insaturation éthylénique de l'hydrocarbure de départ.

7. Produit soufré selon l'une des revendications 1 à 6, caractérisé en ce que le carbonate de guanidine est mis en jeu en une proportion de 3.10⁻³ à 1,2 mole par insaturation éthylénique de l'hydrocarbure de départ.

8. Produit soufré selon l'une des revendications 1 à 7, caractérisé en ce que la réaction est réalisée à une température de 50 à 200°C et sous une pression, allant de la pression atmosphérique jusqu'à 50 bars.

9. Produit soufré selon l'une des revendications 1 à 7, caractérisé en ce qu'il renferme de 20 à 70 % en masse de soufre.

10. Utilisation d'un produit soufré selon des revendications 1 à 9 comme additif dans des compositions lubrifiantes, à une concentration de 0,05 à 20 % en masse.

11. Utilisation d'un produit soufré selon les revendications 1 à 9 comme agent de sulfuration dans la préparation d'un catalyseur de raffinage de produits pétroliers.
